Europäisches Patentamt

European Patent Office

Office européen des brevets

(11) Publication number: **0 165 994**
**B1**

# (12) EUROPEAN PATENT SPECIFICATION

(45) Date of publication of patent specification: **18.07.90**

(51) Int. Cl.⁵: **A 61 K 49/04**

(21) Application number: **85900568.8**

(22) Date of filing: **28.12.84**

(86) International application number:
**PCT/US84/02127**

(87) International publication number:
**WO 85/03004 18.07.85 Gazette 85/16**

## (54) CONTRAST AGENTS AND METHOD OF USE THEREOF.

(30) Priority: **29.12.83 US 566494**

(43) Date of publication of application:
**02.01.86 Bulletin 86/01**

(45) Publication of the grant of the patent:
**18.07.90 Bulletin 90/29**

(84) Designated Contracting States:
**AT BE CH DE FR GB LI LU NL SE**

(56) References cited:
**US-A-2 348 231**

**RADIOLOGY, vol. 49, no. 2, August 1947;
W.E.CHALECKE et al.: "Iodinated organic
compounds as contrast media for radiographic
diagnoses", pp. 131-136**

**RADIOLOGY, vol. 55, July 1950; E.L.PIRKEY et
al.: "Clinical studies of the use of emulsion of
ethyl iodophenylundecylate(pantopaque)", pp.
89-93**

(73) Proprietor: **RUBIN, Daniel Lee
575 South Rengstorff
Mountain View, CA 94040 (US)**

(72) Inventor: **RUBIN, Daniel Lee
575 South Rengstorff
Mountain View, CA 94040 (US)**

(74) Representative: **Goldin, Douglas Michael
J.A. KEMP & CO. 14, South Square Gray's Inn
London WC1R 5EU (GB)**

(56) References cited:
**RADIOLOGY, vol. 82, March 1964; J.G.TEPLICK
et al.: "Experimental studies with a new
radiopaque emulsion", pp. 478-484**

## Description

This invention relates to contrast agents and processes for their use in diagnostic radiology. More specifically, it relates to contrast agents and their use in the gastrointestinal tract.

The use of roentgenographic (X-ray) techniques to assist in the diagnosis of medical problems is well known and long established. For example, the diagnosis of fractures, arthritic conditions and other problems associated with the skeletal system is a routine and highly effective practice. Chest X-rays to provide early detection or confirmation of lung and heart diseases are also routinely done.

The use of X-rays for the purpose of visualizing these organs is facilitated because there are sufficient differences in the densities of air, bone and soft tissue to produce an image on the X-ray film. However, the performance of an X-ray on abdominal organs can be impractical, if not impossible, because the densities of these organs are so similar that a satisfactory image cannot be obtained.

Notwithstanding the fact that an organ may not be visualized on X-ray, in many instances radiological techniques of diagnosis can still be accomplished through the use of contrast agents. Ideally, such an agent, which contains a substance denser than the adjacent tissues is administered to the patient in a manner which causes the agent to be distributed throughout the organ of interest.

Among the organs commonly examined by X-ray using contrast agents is the gastrointestinal (GI) tract. The most widely used contrast agent for the GI tract, but by no means an ideal one, is barium sulfate administered orally or rectally as a suspension. The suspensions, however have limited stability even with stabilizers, have poor palatability and are constipating. Furthermore, in the upper GI tract, the suspensions lack homogeneity, becoming flocculent under the influence of gastrointestinal secretions, and do not adhere well to mucus membranes. Barium sulfate is also highly radio-opaque to X-ray and, as a result, a segment of small bowel or colon underlying another segment is obscured because of inadequate penetration by the X-ray radiation. When given orally, barium sulfate preparations are useless as an aid in examination of the colon as no coating of the mucosa occurs, the barium sulfate forming irregular clumps with fecal material. Furthermore, when administered as an enema, barium sulfate suspensions in the presence of fecal material show the same tendencies to flocculate and to coat the mucoas poorly.

Aqueous solutions of suitable radio-opaque molecules have been proposed as contrast agents for use in the GI tract and ar less constipating than barium sulfate preparations. However, they are even less palatable than barium preparations and have other substantial deficiencies which have prevented them from gaining acceptance for that purpose. For example, they are hypertonic and highly irritating to the GI tract and are even considered to be hazardous to patients in the pediatric age group. They are also poor contrast agents in the GI tract, exhibiting relatively low radio-density which is made worse by osmotic diluation. The solutions, being water soluble, produce little or no mucosal coating as well.

Another class of contract agents used for X-ray studies are oily organic substances containing iodine to confer radioopacity. Among these may be mentioned Ethiodol (the iodine addition product of a mixture of ethyl esters of oleic, linoleic and linolenic acids obtained from poppyseed oil), Lipiodol (the iodine addition product of glyceryl esters of oleic, linoleic and linolenic acids), Agiopaque (ethyliodosterate), the isobutyl ester of diiodobehenolic acid and iodohexadecane. It will be recognized that these agents, but for iodohexadecane, are the product of adding iodine to the olefinic sites of esters of long chain (18—22 carbon atoms) unsaturated fatty acids. These substances have found no application in the GI tract because, being oily, they are not miscible with aqueous GI contents and do not coat the mucosa.

Emulsions of this class of contrast agents have been proposed for use in the GI tract, but pose substantial problems of toxicity. Teplich used emulsions of Ethiodol. See Teplich et al., Radiology, 82, 478 (1964). Reportedly, emulsions in which the particle size had been reduced to less than 0.3 micron gave good contrast visualization in the small intestine of dogs used as test subjects. In those tests, however, 50—70% of the oil was absorbed from the GI tract of the animals. Between 20—57% of the oil was eliminated by kidney function but the balance was stored in the body, posing the threat of a toxic reaction. Teplich et al. performed some clinical trials with human test subjects without reporting any data, but the results were such that the authors concluded that "an emulsion of a completely non-absorbable" radio-opaque substance "might" be ideal for use in the GI tract. On the other hand, the result of emulsifying an oil to improve its performance as a contract agent also reduces its particle size which increases its absorbability and potential toxicity. In any case, emulsions of Ethiodol have not gained acceptance for use in the GI tract notwithstanding the well known shortcomings of barium sulfate preparations.

The use of an aqueous emulsion comprising 50% by volume of iophendylate "sparingly in the examination of the upper gastrointestinal tract" has been reported to produce results no better than that achieved using conventional materials. See Pirkey et al., Radiology, 55, 89, 92 (1950).

From the foregoing, it will be apparent that there has gone unfilled a long felt need for a method of radiological examination of the GI tract which employs a contrast agent that is convenient to use without inflicting discomfort on the patient or posing substantial toxicity problems and which coats the mucosa well, is stable in the GI tract and, at the same time, exhibits satisfactory radioopacity and permits transradiation to permit visualization of underlying bowel segments.

## Summary of the invention

The present invention provides a contrast agent which comprises an aqueous mixture or an aqueous emulsion having up to 45% by volume of an iodinated compound selected from an aliphatic carboxylic acid or a compound convertible, at least in part, in the GI tract to the aliphatic carboxylic acid such as an ester, amide or anhydride, the carboxylic acid having an aliphatic chain, including the carboxyl group, of from 6 to 17 carbon atoms and being but slightly soluble in water.

The contrast agent can be administered orally or rectally for the radiological examination of the GI tract and can be used for the manufacture of a formulation for the diagnosis of conditions of the GI tract.

The mixtures or emulsions of the invention may be administered orally to a patient for radiological examination of the GI tract and X-rays taken as the preparation proceeds through the esophagus, stomach, small intestine and, significantly, the colon (large intestine), an organ which was heretofore been examined only using rectally administered agents. The mixtures or emulsions of the invention may also be administered rectally to a patient for radiologic examination of the colon if desired.

In a preferred embodiment of the invention, the contrast agent is either an aqueous mixture or, an emulsion of iophendylate (commercially available under Trade Marks including Pantopaque, Ethiodon, Myodil and Neurotrast).

Accordingly, it is an object of the present invention to achieve improved contrast visualization of the GI tract.

Another object of the invention is to provide improved contrast agents for oral or rectal administration for visualization of the GI tract.

A more specific object is to visualize the colon by means of orally administered contrast agents.

The manner in which these and other objects are achieved will be apparent from a consideration of the accompanying figures and the following detailed description of the invention.

## Brief description of the figure

Figures 1 to 7 are X-rays taken of the GI tract of a dog after oral administration of contrast agents according to the present invention.

Figures 8 and 9 are X-rays taken of the GI tract of the same animal after oral administration of a barium sulfate suspension for comparison with the method of the present invention.

Figures 10—13 are X-rays taken of the GI tract of a dog using a composition believed to have been known to the prior art.

## Detailed description of the invention

As already indicated, the present invention provides an improved contrast agent in the form of either an aqueous mixture or aqueous emulsion of an iodinated compound selected from aliphatic carboxylic acids or compounds convertible, at least in part, in the GI tract to the aliphatic carboxylic acid, the carboxylic acid having an aliphatic chain, including the carboxylic group, of from 6 to 17 carbon atoms, not including branching, and being only slightly soluble in water. As used herein, the term "slightly soluble" means that the solubility of the iodinated compound is greater than that of Ethiodol but which is not miscible with water in all proportions. Preferably, the solubility of the iodinated compound in water is not greater than about 10% by volume. After either oral or rectal administration of the contrast agent, X-rays are taken at suitable time intervals as the agent passes through the GI tract. Among compounds convertible to the aliphatic acid may be mentioned amides, esters and anhydrides of the aliphatic carboxylic acid.

I have found that good contrast visualization of the GI tract can be obtained according to the present invention, particularly of the small intestine and even of the large intestine by the oral route. While I do not wish to be bound by any particular theory, it appears that the improved results obtained by my invention compared to prior art suspensions of barium sulfate and emulsions of Ethiodol, both of which are substantially insoluble in water, and compared to soluble contrast agents in the form of aqueous solutions are, at least in part, the result of the partial or slight water solubility of the iodinated compounds of the invention. Their slight water solubility permits diffusion of the iodinated compounds into the aqueous phase of the intestinal mucus and secretions which coat the mucosa without the need to form emulsions of very small particle size as is necessary with less polar, more hydrophobic and insoluble substances such as Ethiodol which coat well only when finely dispersed. Their low solubility also prevents their osmotic dilution as occurs with soluble contrast agents without substantially impairing their ability to coat the mucosa.

The slight water solubility of the compounds permits their formulation into aqueous mixtures of large particle size, by simple shaking of such compounds with water since there is no need to reduce the particles to a very small size in order to achieve coating of the GI tract as is required, for example, with Ethiodol. Because larger particle sizes can be used, the absorption of the iodinated compound is reduced which reduces the possibility of toxic effects being observed.

It also appears that the aliphatic carboxylic acid structure of the iodinated compounds of the invention facilitates their packaging into bile salt micelles in the intestine which aid their transport into the mucus layer. It further appears that these iodinated compounds exhibit an affinity for the mucus and intestinal epithelium since, as will be demonstrated hereinafter, the iodinated compound persists in the mucus of the colon for up to 72 hours after oral administration and even after evacuation of all colonic content. This property is not exhibited by any contrast agent used heretofore in the GI tract.

Aliphatic iodinated compounds used in the contrast agent can be obtained by the addition of iodine to unsaturated bonds on the aliphatic chain by conventional techniques. Iodine can also be incorporated by attaching an iodinated aromatic side chain to the molecule. The latter compounds are preferred because the iodine, being bound to an aromatic group, is not physiologically labile during metabolism and is excreted with the aromatic portion of the molecule, further reducing the possible toxic side effects. Aliphatically bound iodine, on the other hand, can be released by deiodination and result in a toxic reaction.

Presently preferred iodinated compounds are aliphatic carboxylic acids, or compounds convertible, at least in part, to carboxylic acids in the GI tract having 6—17 carbon atoms in the aliphatic chain, not including branching, including the carboxyl group, and having an iodinated aromatic substituent on the chain. Such compounds and the method of their preparation are described in U.S. Patent 2,348,231, the disclosure of which is incorporated herein by reference. A particularly preferred iodinated compound in this class is iophendylate, a mixture of a major portion of ethyl 10-(p-iodophenyl)undecylate with a minor portion of ethyl 11-(p-iodophenyl)undecylate.

Generally, the class of preferred compounds is that having the formula $(I)_n$—X—R—COR' wherein the R is an aliphatic chain of from 5 to 16 carbon atoms exclusive of branching, X is an aromatic group which one or more iodine atoms are attached, n is an integer of from 1—3 per aromatic nucleus and R' is a hydroxy group, a halogen atom or an alkoxy, amino or acyloxy group. While a variety of alcohols can be used to make the ester, alcohols such as ethyl and glyceryl are preferred since, if liberated in the GI tract by a saponification process, they are relatively benign substances which the body tolerates well. Preferably X is a phenyl group.

As indicated, the iodinated compound can be formulated as an aqueous mixture or as an emulsion. In presently preferred compositions, the amount of iodinated compound does not exceed about 45% by volume of an emulsion or mixture. When it is desired to use an emulsion, the emulsifying agent can be selected from commercially available agents. Some, however, are to be preferred over others and the most satisfactory ones can be selected by simply screening available emulsifiers. For example, pectin seems to inhibit gastric emptying and is undesirable for that reason. Others form emulsions which lack sufficient stability in the GI tract for optimum results. Among these may be mentioned Myrj 45, gelatin and mixtures of Myrj 45 and Between 20. A presently preferred emulsifier is Dow Corning Medical Antifoam AF, a composition of 30% poly-dimethylsioloxane and silica aerogel, 14% stearate emulsifiers, and 0.075% sorbic acid, the balance being water.

When it is desired to use an aqueous mixture the iodinated compound can be dispersed in water by simple shaking with or without commercially available additives.

Other preferred formulations are emulsions or aqueous mixtures prepared with stabilizers including but not limited to biopolymers such as Emulsan (Petroferm USA), a lipoheterpoly-saccharide comprising a backbone of D-galactosamine and amino-uronic acid having fatty acid and fatty ester side chains of length ranging from $C_{10}$ to $C_{18}$, the polymers being completely N-acylated and partially O-acylated.

The emulsion or mixture may be administered orally and X-rays taken at the desired intervals of the esophagus, stomach, small intestine and the colon. The emulsion or mixture may also be administered rectally for visualization of the colon. If radiological examination of the stomach is desired, a buffering agent should be given first to neutralize stomach acid as better coating is obtained in a non-acidic medium.

The following emulsions or mixtures or iophenydylate (Pantopaque) have been found to provide particularly satisfactory results in the method of the invention.

1. 36 ml Pantopaque
   120 ml water
   50 ml Dow Corning Medical Antifoam AF
2. 36 ml Pantopaque
   170 ml water
3. 150 ml Pantopaque
   50 ml water
   150 ml Dow Corning Medical Antifoam AF.

All of the formulations were prepared by mixing the ingredients in a cup and shaking the mixture for 1 minute before oral administration. In the cases of formulations 1 and 3, stable emulsions were formed. A simple mixture resulted from shaking formulation 2.

Each of these formulations were orally administered on different occasions to the same dog (approximate weight, 20 kilograms) and X-rays taken of the GI tract with the dog in the right lateral position. The X-rays were taken from a distance of 40 inches to the film plane, at 100 kilovolts and 30 milli-amperes seconds (300 milliamps, 0.1 sc.). Prior to administration of the contrast agent formulation, the dog was anesthetic was 5 cc administered intravenously. At this level the dog maintained blink reflex but tolerated a stomach tube through which the contrast agent was admitted to the stomach. Anaesthesia was maintained by the periodic administration of 1/2 cc of the Surital solution.

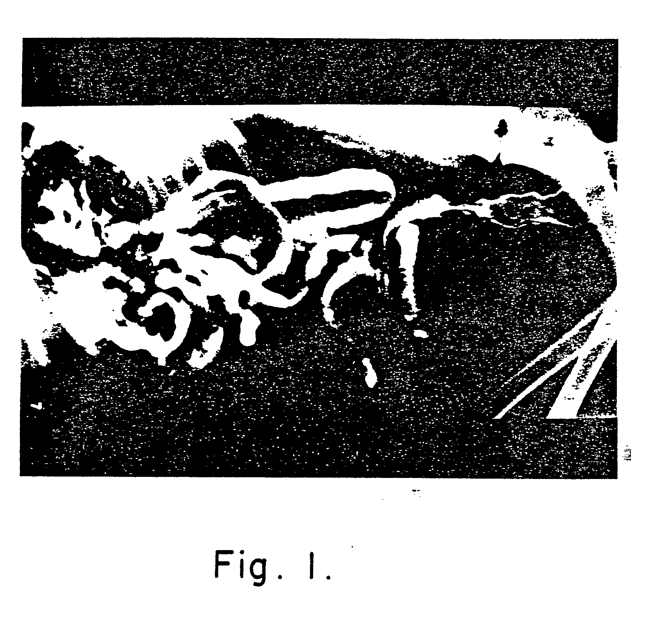

Figures 1, 2 and 3, respectively, are X-rays taken after administration of formulation 1, at 30 minutes, 24 hours, and 48 hours.

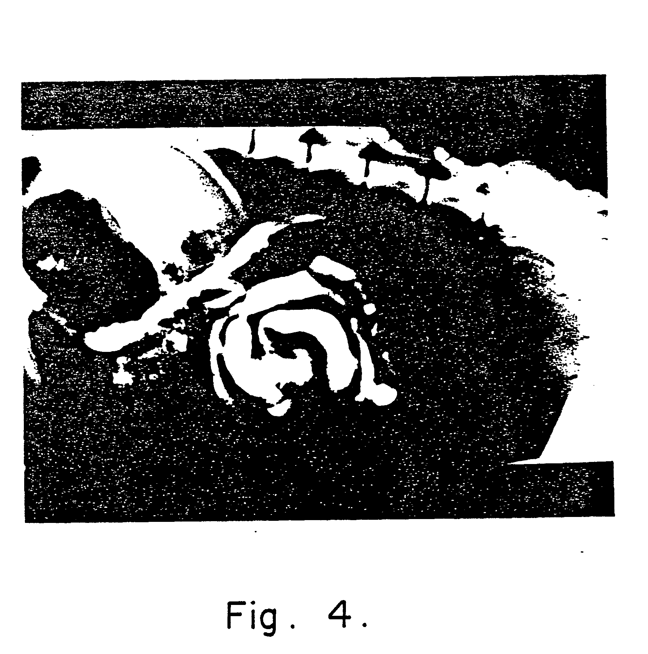

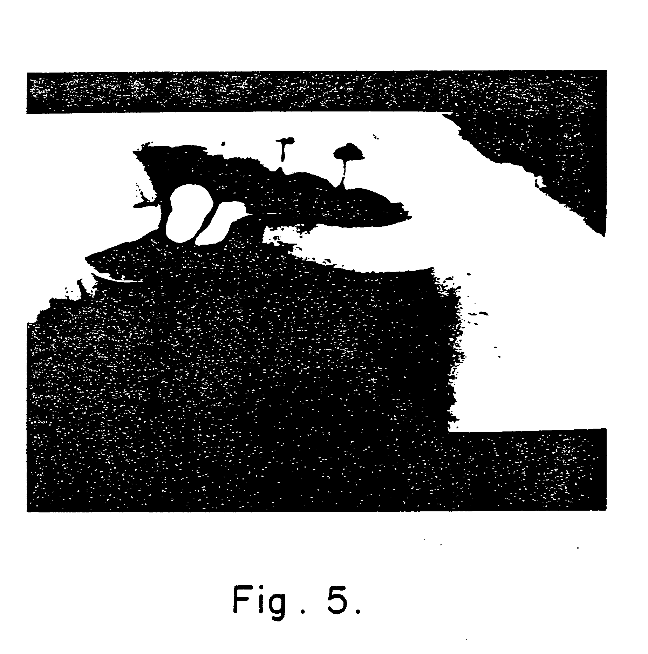

Figures 4 and 5, respectively, are X-rays taken after administration of formulation 2 at 30 minutes and 24 hours.

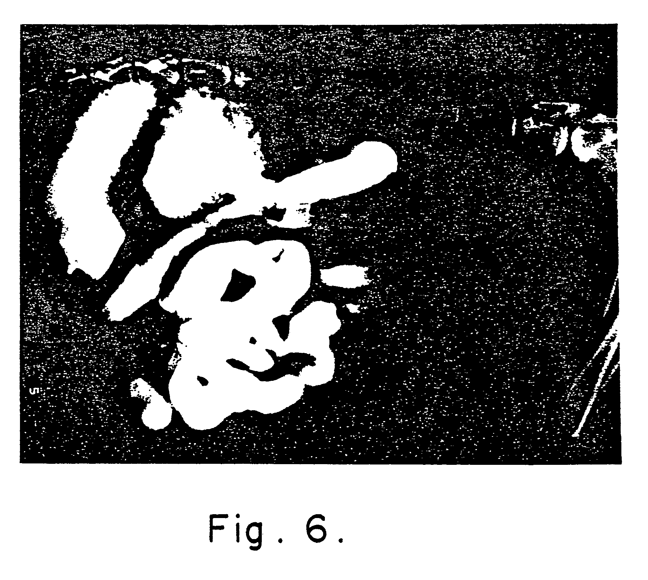

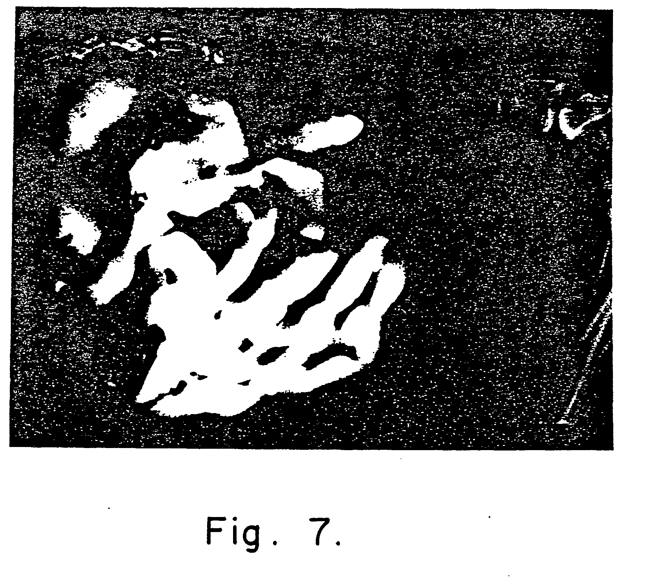

Figures 6 and 7, respectively, are X-rays taken after administration of formulation 3 at 30 minutes and 45 minutes.

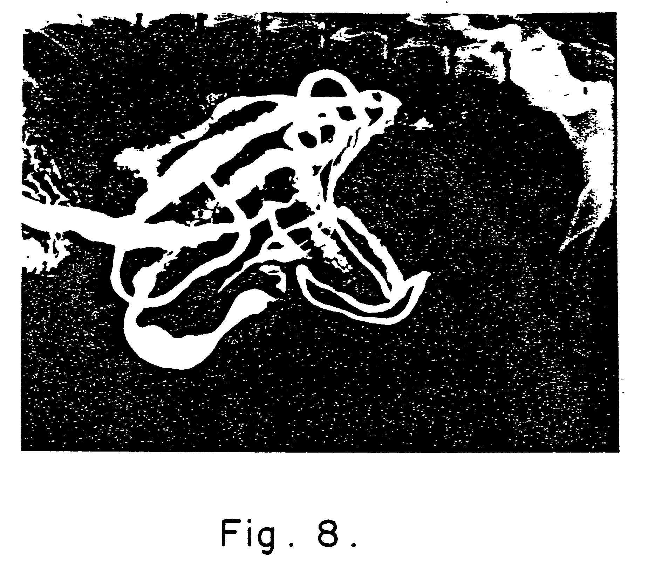

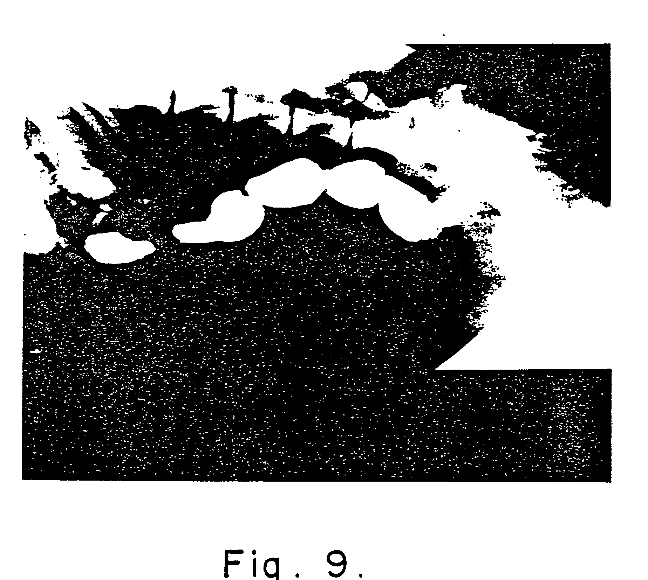

Figures 8 and 9, respectively, are X-rays taken after administration of an aqueous suspension comprising 40% by weight of barium sulfate at 30 minutes and 24 hours for the purpose of comparison with the present invention.

The advantages of the present invention over radiological examination of the GI tract using conventional barium sulfate suspensions is clear from a consideration of Figs. 1—9. Thus, using the emulsions of formulations 1 and 3, excellent contrast visualization of the small intestine with homogeneous opacity in the lumen is obtained which is not affected by intestinal secretions. (Figs. 1, 6 and 7). Furthermore, excellent mucosal coating is observed and over-lapping intestinal loops show an excellent transradiation effect which permits visualization of each loop. (Figs. 1, 6 and 7). In Fig. 7, it can be seen that excellent air contrast visualization of the small bowel is obtained with a thick and homogeneous layering of contrast medium on the mucosal surface. In Fig. 6, it can further be seen that the emulsion produces a coating of the gastric mucosa.

Even simple mixtures of water and iophendylate produced excellent mucosal coating and contrast visualization of the small intestine. (Compare Figs. 1 and 4).

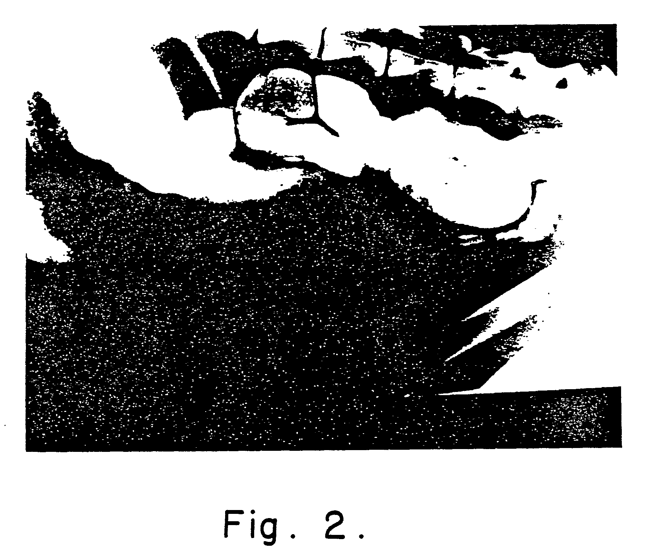

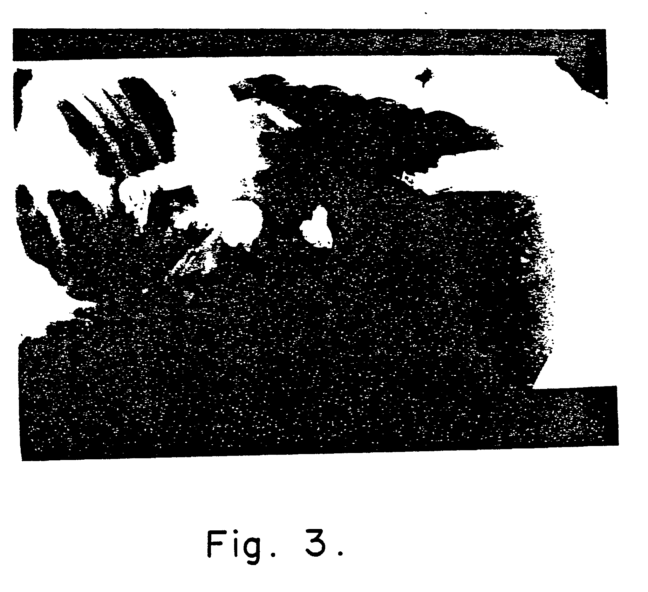

After 24 hours from ingestion, both emulsions and mixtures of iophendylate show persistence of mucosal coating and contrast visualization in the terminal ileum and colon, (Figs. 2 and 5). Partial contrast visualization is retained even after 48 hours (Fig. 3). Although not shown, some persistence of coating has been observed even after 72 hours.

By contrast, X-rays taken after administration of barium sulfate are substantially inferior to those obtained with both emulsions and simple mixtures of iophendylate. They do not demonstrate the occurrence of transradiation through overlapping loops (Fig. 8) and no contrast visualization of the colon is obtained since the barium sulfate has only formed irregular clumps with the fecal material (Fig. 9).

No substantial adverse effects on the test subject were observed after administration of the emulsions or mixtures of iophendylate. In fact, no adverse reaction at all was observed when the oil and water mixture was administered, the dog becoming active and alert after the effects of the anaesthetic wore off. In the case of the emulsions, occasionally diarrhoea was observed and mild lethargy persisted for up to 48 hours. No other adverse effects were noted and at least some of the effects noted may have been caused by the anaesthetic.

Although standard X-rays were obtained using the formulations described above, fluoroscopy or cineradiography techniques can be used as well.

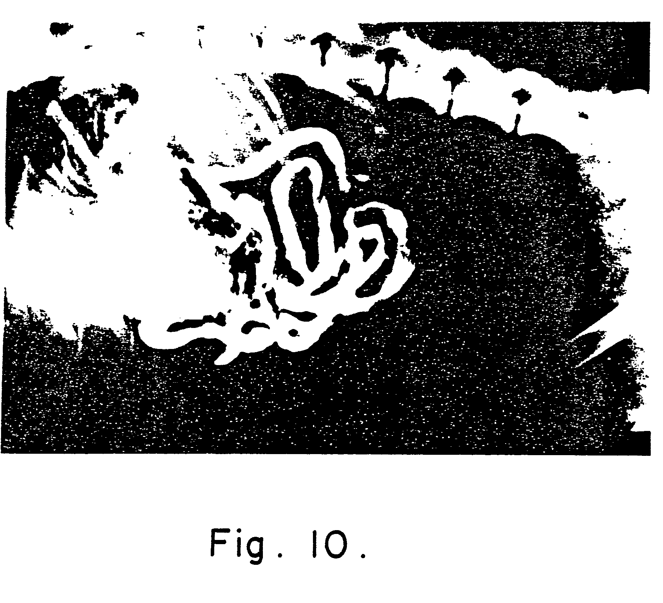

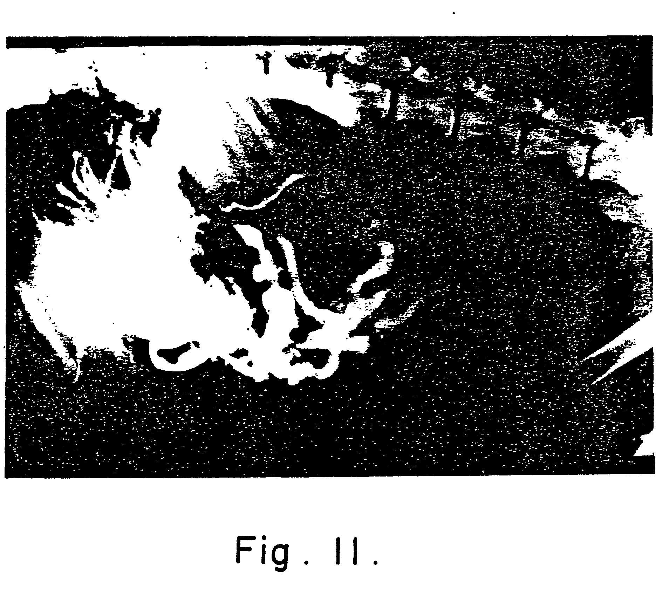

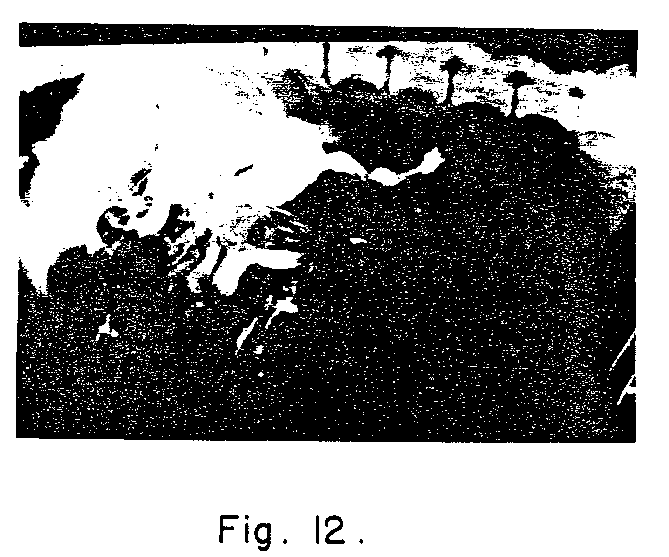

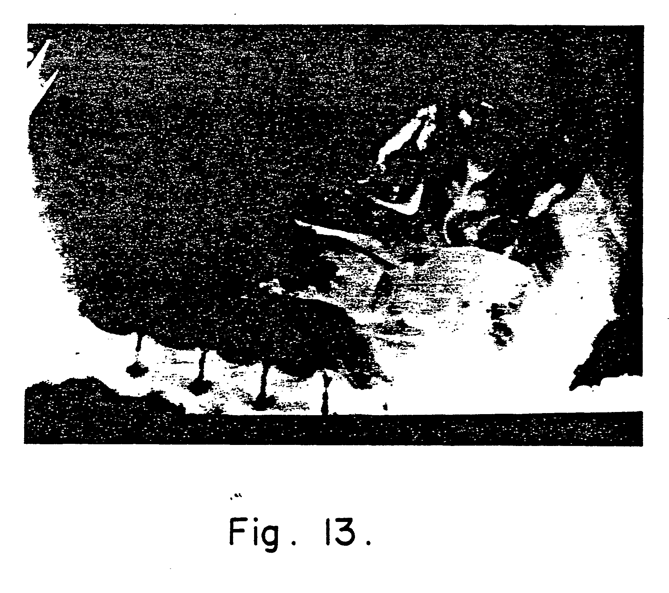

As noted above, Pirkey et al., In *Radiology*, 55, 89 (1950) report use of an aqueous emulsion comprising 50% by volume of Pantopaque "sparingly" for upper GI tract examination with no better results than with conventional materials. They reference having a formulation described by Cholecke et al. *Radiology*, 49, 131 (1947). That composition consisted of Pantopaque, water and about 1% of Igepon % as a surface active agent. Pirkey et al. did not reproduce or even describe the X-rays taken which were the basis for their conclusions. Figures 10—13 are radiographs taken of the GI tract of a dog taken 10, 15, 45 and 55 minutes, respectively, after administration of an emulsion prepared according to the Cholecke formula used by Pirkey et al.

The radiographs taken after 10 and 15 minutes, when about 2/3 of the small intestine can be visualized, are substantially as described by Pirkey. The X-rays taken after 45 and 55 minutes to visualize the entire small intestine show, however, very poor visualization as the emulsion has begun to breakdown, apparently because of concentration effects. Furthermore, the emulsion is apparently quite toxic to the animal since it was still lethargic for a full 24 hours after administration.

These results stand in marked contrast to those observed in connection with the formulations used to obtain Figs. 1—7 which are described above. For example, Fig. 7 shows good mucosal coating and contrast visualization of the small intestine 45 minutes after administration, with no sign of emulsion breakdown. Furthermore, in those cases the test animals recovered rapidly from the treatment regimen.

From the foregoing, it will be apparent that the present invention provides a greatly improved contrast agent for radiologic examination of the GI tract compared to accepted prior art practices. Unexpectedly, even satisfactory visualization of the colon can be obtained by oral administration of a contrast agent of this invention eliminating, in many cases at least, the need for cleansing the colon using a laxative or enema. Furthermore, the procedure is simpler than conventional methods for visualizing the colon which require extremely uncomfortable administration of barium by enema after the cleansing procedure. Heretofore, no orally administered contrast agent has been available for visualization of the colon. The agents of the present invention, however, permit a full GI tract examination using an orally administered agent.

That variations from the preferred embodiments described above will also be useful will be apparent to those skilled in the art. Therefore, the present invention is to be considered limited only by the appended claims.

## Claims

1. An aqueous emulsion or mixture comprising up to 45% by volume of an iodinated compound which is an aliphatic carboxylic acid of from 6—17 carbon atoms in the aliphatic chain including the carboxyl group, or derivative thereof which is converted to the carboxylic acid in the gastro-intestinal tract, the iodinated compound being slightly soluble in water, for use in a method of diagnosis on the human or animal body.

2. An emulsion or mixture according to claim 1 in which the iodinated compound has an aromatic substituent to which the iodine is bound.

3. An emulsion or mixture according to claim 1 or 2 in which the iodinated compound is of formula $(I)_nX—R—COR'$ in which X is an aromatic group to which one or more iodine atoms are attached, n is an integer of from 1 to 3 per aromatic nucleus, R is an aliphatic group of 5 to 16 carbon atoms exclusive of branching, and R' is a hydroxy group, a halogen atom or an alkoxy, amino or acyloxy group.

4. An emulsion or mixture according to claim 3 in which the iodinated compound is iophendylate.

5. An emulsion or mixture according to any one of claims 1 to 4 in a form suitable for oral administration.

6. An emulsion or mixture according to any one of claims 1 to 4 in a form suitable for rectal administration.

7. Use of an emulsion or mixture according to any one of claims 1 to 6 for the manufacture of a formulation for the diagnosis of conditions of the gastrointestinal tract.

8. A contrast agent for visualization of the GI tract comprising an aqueous emulsion or mixture comprising up to 45% by volume of an iodinated aliphatic carboxylic acid of from 6—17 carbon atoms in the aliphatic chain including the carboxyl group or other compound which is a derivative thereof which is converted to a carboxylic acid in the GI tract, the iodinated compound being only slight soluble in water.

9. A contrast agent according to claim 8, wherein the compound has an aromatic substituent to which the iodine is bound.

10. A contrast agent according to claim 9 wherein the iodinated compound is of the formula $(I)_nX—R—COR'$ wherein X is an aromatic group to which one or more iodine atoms are attached, n is an integer of from 1—3 per aromatic nucleus, R is an aliphatic group of from 5 to 16 carbon atoms exclusive of branching, and R' is a hydroxy group, a halogen atom or an alkoxy, amino or acyloxy group.

11. A contrast agent according to claim 10, wherein the iodinated compound is iophendylate.

## Patentansprüche

1. Wässerige Emulsion oder Mischung umfassend bis zu 45 Vol. % einer iodierten Verbindung, die eine aliphatische Carbonsäure mit 6 bis 17 Kohlenstoffatomen in der aliphatischen Kette, einschließlich der Carboxylgruppe, darstellt, oder ein Derivat davon, das im Gastrointestinaltrakt in die Carbonsäure überführt wird, und wobei die iodierte Verbindung in Wasser schwach löslich ist, zur Verwendung in einem Verfahren zur Diagnose des menschlichen oder tierischen Körpers.

2. Emulsion oder Mischung nach Anspruch 1, dadurch gekennzeichnet, daß die iodierte Verbindung einen aromatischen Substituenten besitzt, an den das Iod gebunden ist.

3. Emulsion oder Mischung nach Anspruch 1 oder 2, dadurch gekennzeichnet, daß die iodierte Verbindung die Formel $(I)_nX—R—COR'$ besitzt, worin X eine aromatische Gruppe ist, an die ein oder mehrere Iodatome gebunden sind, n eine ganze Zahl von 1 bis 3 pro aromatischem Kern darstellt, R eine aliphatische Gruppe mit 5 bis 16 Kohlenstoffatomen, ausschließlich eine Verzweigung, ist, und R' eine Hydroxygruppe, ein Halogenatom oder eine Alkoxy-, Amino- oder Acyloxygruppe ist.

4. Emulsion oder Mischung nach Anspruch 3, dadurch gekennzeichnet, daß die iodierte Verbindung Iophendylat ist.

5. Emulsion oder Mischung nach einem der Ansprüche 1 bis 4 in einer zu oralen Verabreichung geeigneten Form.

6. Emulsion oder Mischung nach einem der Ansprüche 1 bis 4 in einer zu rektalen Verabreichung geeigneten Form.

7. Verwendung einer Emulsion oder Mischung nach einem der Ansprüche 1 bis 6 zur Herstellung einer Formulierung zur Diagnose des Zustandes des Gastrointestinaltraktes.

8. Kontrastmittel zur Darstellung des GI-Traktes, umfassend eine wässerige Emulsion oder Mischung, die bis zu 45 Vol. % einer iodierten aliphatischen Carbonsäure mit 6 bis 17 Kohlenstoffatomen in der aliphatischen Kette, einschließlich der Carboxylgruppe, enthält, oder eine andere Verbindung, die ein Derivat davon ist, das im GI-Trakt zu einer Carbonsäure überführt wird, und wobei die iodierte Verbindung in Wasser nur schwach löslich ist.

9. Kontrastmittel nach Anspruch 8, dadurch gekennzeichnet, daß die Verbindung einen aromatischen Substituenten besitzt, an den das Iod gebunden ist.

10. Kontrastmittel nach Anspruch 9, dadurch gekennzeichnet, daß die iodierte Verbindung die Formel $(I)_nX—R—COR'$ besitzt, worin X eine aromatische Gruppe ist, an die ein oder mehrere Iodatome gebunden sind, n eine ganze Zahl von 1 bis 3 pro aromatischem Kern darstellt, R eine aliphatische Gruppe mit 5 bis 16 Kohlenstoffatomen, ausschließlich Verzweigungen, ist, und R' eine Hydroxygruppe, ein Halogenatom oder ein Alkoxy-, Amino- oder Acyloxygruppe.

11. Kontrastmittel nach Anspruch 10, dadurch gekennzeichnet, daß die iodierte Verbindung Iophendylat ist.

## Revendications

1. Emulsion ou mélange aqueux comprenant jusqu'à 45% en volume d'un composé iodé qui est un acide carboxylique aliphatique, comportant 6—17 atomes de carbone dans la chaîne aliphatique, y compris le groupe carboxylique, ou un de ses dérivés, qui est converti en cet acide carboxylique dans le tractus gastro-intestinal, le composé iodé étant peu soluble dans l'eau, pour être utilisé dans un procédé de diagnostic relatif au corps humain ou animal.

2. Emulsion ou mélange selon la revendication 1, dans lequel le composé diodé a un substituant aromatique auquel l'iode est lié.

3. Emulsion ou mélange selon la revendication 1 ou 2, dans lequel le composé iodé répond à la formule (I)$_n$X—R—COR', où X est un groupe aromatique auquel sont liés un ou plusieurs atomes d'iode, n est un nombre entier de 1 à 3 par noyau aromatique, R est un groupe aliphatique de 5 à 16 atomes de carbone, exempt de ramification, et R' est un groupe hydroxyle, un atome d'halogène ou un groupe alcoxy, amino ou acyloxy.

4. Emulsion ou mélange selon la revendication 3, dans lequel le composé iodé est l'iophendylate.

5. Emulsion ou mélange selon l'une quelconque des revendications 1 à 4, sous une forme adaptée pour l'administration orale.

6. Emulsion ou mélange selon l'une quelconque des revendications 1 à 4, sous une forme adaptée pour l'administration rectale.

7. Utilisation d'une émulsion ou mélange selon l'une quelconque des revendications 1 à 6, pour la fabrication d'une formulation pour le diagnostic de l'état du tractus gastro-intestinal.

8. Agent de contraste pour la visualisation du tractus GI, comprenant une émulsion ou mélange aqueux contenant jusqu'à 45% en volume d'un acide carboxylique aliphatique iodé, comportant 6—17 atomes de carbone dans la chaîne aliphatique, y compris le groupe carboxyle, ou un autre composé qui est un de ses dérivés, qui est converti en cet acide carboxylique dans le tractus gastro-intestinal, le composé iodé n'étant que peu soluble dans l'eau.

9. Agent de contraste selon la revendication 8, dans lequel le composé comprend un substituant aromatique auquel est lié l'iode.

10. Agent de contraste selon la revendication 9, dans lequel le composé iodé répond à la formule (I)$_n$X—R—COR', où X est un groupe aromatique auquel sont liés un ou plusieurs atomes d'iode, n est un nombre entier de 1 à 3 par noyau aromatique, R est un groupe aliphatique de 5 à 16 atomes de carbone, exempt de ramification, et R' est un groupe hydroxyle, un atome d'halogène ou un groupe alcoxy, amino ou acyloxy.

11. Agent de contraste selon la revendication 10, dans lequel le composé iodé est l'iophendylate.

Fig. I.

Fig. 2.

Fig. 3.

Fig . 4 .

Fig . 5.

Fig. 6.

Fig. 7.

Fig. 8.

Fig. 9.

Fig. 10.

Fig . II .

Fig. 12.

Fig. 13.